# EUROPEAN PATENT SPECIFICATION

(11) **EP 1 250 088 B1**
(45) Date of publication and mention of the grant of the patent: **27.12.2006**
(21) Application number: 01905117.6
(22) Date of filing: 29.01.2001
(51) Int. Cl.: A61B 5/103, A61B 5/00

(54) **DETECTION AND QUANTIFICATION OF JOINT AND TISSUE INFLAMMATION**
ERFASSUNG UND QUANTIFIZIERUNG VON GELENK- UND GEWEBEENTZÜNDUNGEN
DETECTION ET QUANTIFICATION DE L'INFLAMMATION D'ARTICULATIONS ET DE TISSUS

(30) Priority: 29.01.2000 US 178872 P
(43) Date of publication of application: 23.10.2002
(73) Proprietor: Thomson, Paul E., Cincinnati, OH 45247-7422 (US)
(72) Inventor: Thomson, Paul E., Cincinnati, OH 45247-7422 (US)
(74) Representative: James, Anthony Christopher W.P.
(86) International application number: PCT/US2001/002660
(87) International publication number: WO 2001/054581

(56) References cited:
- WO-A-90/14042
- WO-A-99/66838
- US-A- 3 970 074
- US-A- 4 275 741
- US-A- 4 445 516
- US-A- 5 025 796
- US-A- 5 588 440

## Description

### Technical Field

The subject invention relates to medical method and apparatus and more particularly to apparatus and method for the detection and quantification of joint and tissue inflammation.

### Background of the Invention

Most people by age 50 develop some degree of arthritis. Usually, this takes the form of degenerative joint disease, or osteoarthritis that eventually afflicts nearly everyone by the time they are 65 years old. Another form of arthritis, rheumatoid arthritis, affects approximately 1% of the population with a prevalence that approaches 2% of males and 5% of females by age 65. When lupus, gout, infectious processes, metabolic processes, toxins, cancers, and the more than 100 other types of arthritis are added to these numbers, nearly everyone, if they live long enough, will suffer joint pain and arthritis. The ultimate prevalence of joint diseases is further increased by fractures, athletic injuries, neuromuscular disorders, and congenital deformities.

Common to all of the various forms of joint diseases is the presence of inflammation. All forms of inflammation, from any cause, are characterized by five cardinal signs or manifestations comprising redness, swelling, heat, pain, and the loss of function of the involved tissue. The presence of inflammation in the involved joint or tissue indicates the presence of injury or disease, while the amount of inflammation in the injured, deformed, or arthritic joint or tissue is directly proportional to the amount of damage or disease in that joint and is inversely proportional to the degree of healing in the same joint. Accordingly, the physician or surgeon treating such musculoskeletal problems is constantly trying to detect any inflammation, and, if present, to assess the degree of this inflammation in order to determine whether disease or injury is occurring, how much disease or damage is present, and whether the problem is progressing or healing.

Various methods and equipment have been developed for assessing the presence or absence of inflammation in joints and other tissues. Such methods and equipment include performing a physical examination of the involved tissue; blood tests, such as erythrocyte sedimentation rate or C-reactive protein level; radiographic tests, such as plain X-rays or magnetic resonance imaging (MRI); and research procedures, such as thermography.

In performing a physical examination, the physician begins the method of assessing the presence or absence of inflammation by asking such questions as which joints or tissue areas are causing pain, how much pain is present and how often is the pain present. The physician will then perform a physical examination to determine if there is any redness, warmth, swelling, or stiffness in these areas, and to see if the patient is experiencing any limitation or loss of function. This method of questioning and physical examination of the patient, however, does not reliably establish the presence of inflammation. For example, redness over an area of pain could be a result of an overlying skin rash rather than inflammation or stiffness may be a result of a nearby muscle spasm as opposed to inflammation. Further, this method results in an assessment that is only crudely and inconsistently quantifiable. For example, the physician might ask, "If zero equals no pain and your pain level was a 10 out of 10 when you were first treated, at what level is your pain currently?" Such clinometric scales are generally inconsistent and results will vary depending, among other factors, on the manner in which the questions were asked, the physician asking the questions and the day and time the patient was questioned. Accordingly, such assessments are highly subjective and ultimately have no definite correlation with the actual degree of inflammation.

In addition to determining the presence of inflammation, the physical examination may also provides some indication of the presence and degree of inflammation. The physician or surgeon, for example, may gently squeeze or palpate the involved joint or tissue to detect swelling, warmth, or tenderness. Even if the physician detects some amount of joint or tissue swelling by palpation, there is no precise and reproducible way of determining whether the swelling represents newly inflamed tissue or just residual thickened and scarred tissue from previous, now quiescent, inflammation. Detection of skin warmth is useful but cannot be quantified by simple palpation. The determination of tenderness is also useful but it only inconsistently correlates with actual inflammation. For example, the joint or tissue could be tender secondary to conditions that are not related to inflammation, such as poor circulation, diabetic-related or other causes of local nerve damage, foreign bodies, and other causes. The degree of tenderness is often assessed using a clinometric scale and has the same drawbacks as previous mentioned. The range of motion of a joint or muscle represents that tissue's functional state. Typically, the method of measuring the range of motion of a joint or muscle involves holding a plastic protractor next to the joint while the patient tries to move the joint or muscle in question. Unfortunately, problems of accuracy and reproducibility arise due to placement and stability of the protractor. It has been found that results of testing will vary even between tests performed by the same examiner on the same patient.

The method of performing blood tests to assess the presence and degree of inflammation typically involves testing of erythrocyte sedimentation rate or C-reactive protein levels. Unfortunately, such tests are nonspecific and indicate changes taking place throughout the patient's body rather than to inflammatory changes taking place in localized areas, such as a knee or finger. Further, the results of the blood tests are uniformly subject to a wide variety of physical conditions and ailments that are not related to inflammation. For example, the blood count in a patient having significant arthritis or other forms of inflammatory disease may show elevations of the white blood cells or of other blood cells called "platelets." However, the white cell count can be elevated due to many conditions, such as leukemia, allergies, drug reactions, and numerous illnesses that have little or no relationship with inflammation. Similarly, the platelet count may be increased by noninflammatory conditions such as an iron deficiency or cancer. In addition, in many inflammatory conditions, such as rheumatoid arthritis, such cell counts typically show no elevation.

In the performance of another type of blood test commonly employed to detect inflammation, the erythrocyte sedimentation rate (ESR), the patient's anticoagulated blood sample is placed in a vertical glass or plastic capillary tube. After sitting for one hour, the height of the column of red blood cells that have settled to the bottom of the tube is measured. In general, the taller the column of red cells, the more inflammation exists in the patient's body. Unfortunately, this method has numerous problems. Similarly to the white blood cell and platelet tests mentioned above, the ESR may not show any elevation, even in the presence of clearly clinically detectable inflammation. This lack of elevation, or in some cases elevated levels not caused by inflammation, may be due to various conditions including anemia, sickled blood cells, bone marrow cancers, and diabetic kidney disease. Further, as with blood count tests, such methods only indicate the possibility of inflammation and reflect a systemic rather than a local situation.

Another blood test used in detecting inflammation involves the measurement of a patient's C-reactive protein level (CRP). Like the other blood tests used for detecting inflammation, CRP suffers from the lack of sensitivity, lack of specificity, and numerous confounding factors.

As a result of the numerous difficulties associated with the various methods of assessing inflammation using blood tests and since these methods do not include measurement of the five above-mentioned markers of inflammation, such methods are indirect at best and only useful in obtaining adjuncts for detecting and quantifying inflammation.

Methods for detecting inflammation using plain radiographs, such as plain X-rays, have proved to be inadequate. It has been found that if a patient has swelling capable of being shown in a X-ray, the swelling level would also be clinically evident on physical examination thereby rendering a X-ray unnecessary. In addition, any shadowy outline of a soft tissue bulge around a bone or joint indicating possible inflammation would be extremely nonspecific and may be produced by conditions that are unrelated to inflammation, such as obesity.

Methods using computerized tomography (CT) scans and magnetic resonance imaging (MRI) scans are performed occasionally to observe swelling in various tissues. Such methods, however, are relatively expensive, may be insensitive to very mild degrees of inflammation, and generally do not detect forms of inflammation that involve only minimal or no swelling. Additionally, the CT scan uses ionizing radiation. Further, an MRI cannot be used for individuals having pacemakers or metallic implants located near the subject area. Another problem with using CT and MRI scans is the difficulty of interpreting the results of the scans making them unsuitable for routine outpatient testing in a typical medical office or clinic.

Methods of detecting inflammation using thermography or infrared photography have been used to try to detect inflammation. Unfortunately, however, thermography is relatively insensitive to mild degrees of inflammation; it cannot detect any of the signs of inflammation except warmth; it is difficult to reproduce due to changes in regional blood flow, for example caused by emotional states; and it is relatively expensive and technically difficult, requiring the patient to be unclothed and placed in a climate-controlled room where ambient temperature and humidity are assiduously constant and stabilized.

US-A-5588440 describes a method and apparatus for locating and assessing soft tissue lesions. The apparatus comprises a probe having means for measuring the moisture content of the skin and a stethoscope for listening to the sound produced during massage of the skin. The probe may optionally also include means for measuring the temperature of the skin.

Accordingly, a need exists for a relatively inexpensive, reliable, reproducible, easy to use or perform, non-invasive apparatus for the detection and quantification of joint and tissue inflammation that may be used in medical offices, clinics, sports and training facilities, and the like.

The present invention provides an apparatus for detecting and quantifying inflammation of a joint or tissue area comprising: a sensing component for obtaining quantitative, non-human-observer measurements of at least two of the cardinal signs of inflammation; a device for storing the measurements; a display device for displaying the measurements; and means for analyzing collected measurements and generating an overall inflammation score.

In a preferred embodiment of the invention, the apparatus for the detection and quantification of joint and tissue inflammation includes a device for determining the amount of loss of function of the joint, selected from a group of spatial orienting and localizing detectors.

In a preferred embodiment of the invention, the apparatus for the detection and quantification of joint and tissue inflammation includes a device for measuring the pain threshold and tolerance of the joint or tissue, selected from the group of pain detecting devices.

In another preferred embodiment of the invention, the apparatus for the detection and quantification of joint and tissue inflammation includes a sensing component which operates to detect swelling via generating measurements of the surface or three-dimensional or cross-sectional or compete spatial models or images of the joint or tissue area.

In another preferred embodiment of the invention, the apparatus for the detection and quantification of joint and tissue inflammation includes a color analyzing device for analyzing the color of the subject joint or tissue comprising a light- or gloss- sensitive device, selected from the group of colorimeters, spectrophotometers, or gloss-meters.

In another preferred embodiment of the invention, the apparatus for the detection and quantification of joint and tissue inflammation includes a temperature measuring device for measuring the temperature of the joint or tissue and is a mechanical contact, optical, laser-based, thermistor type, thermometer, thermographic type, infrared-based, or surface electrical conductance-resistance based temperature measuring device.

A primary object of this invention, therefore, is to provide an apparatus for the detection and quantification of joint and tissue inflammation.

Another primary object of this invention is to provide a relatively inexpensive apparatus for the detection and quantification of joint and tissue inflammation.

Another primary object of this invention is to provide a relatively reliable apparatus for the detection and quantification of joint and tissue inflammation.

Another primary object of this invention is to provide an apparatus that produces reproducible results for the detection and quantification of joint and tissue inflammation.

Another primary object of this invention is to provide a relatively easy to use apparatus for the detection and quantification of joint and tissue inflammation.

Another primary object of this invention is to provide a safe and a noninvasive apparatus for the detection and quantification of joint and tissue inflammation.

Another primary object of this invention is to provide an apparatus for the detection and quantification of joint and tissue inflammation that may be used and performed in medical offices, clinics, sports and training facilities, and the like.

These and other objects and advantages of the invention will be apparent from the following description and the accompanying drawings, in which:
FIG. 1 is a schematic view of the apparatus for the detection and quantification of joint and tissue inflammation of the present invention;
FIG. 2 is a schematic view of the surface scanning element of the sensing component of the apparatus for the detection and quantification of the subject joint of tissue of FIG. 1;
FIG. 3 is a schematic view of the sensing component of the apparatus for the detection and quantification of inflammation of FIG. 1, the sensing component having a distance or spatial measuring device or imaging device to measure the distance between the surface scanning element and the spatial or dimensional characteristics or image of the location being scanned of the subject joint and tissue of **FIG. 1**;
**FIG. 4** is a schematic view of the sensor of the sensing component of the apparatus for the detection and quantification of inflammation of **FIG. 1** showing a support structure and guide track for supporting and directing the motion of the sensor unit;
**FIG. 5** is a schematic view of the imaging system of the apparatus for the detection and quantification of inflammation of **FIG. 1** having mechanical calipers which are applied directly to the patient to measure the cross-sectional dimensions or elevations of the joint or tissue;
**FIG. 6** is a schematic view of the sensing component of the apparatus for the detection and quantification of inflammation of **FIG. 1** showing the surface scanning element having a optical-, gloss-, or temperature- sensitive sensor; and
**FIG. 7** is a schematic view of the sensing component of the apparatus for the detection and quantification of inflammation of **FIG. 1,** the sensing component having a probe for applying a stimuli to the patient at a location to determine pain tolerance and threshold.

### Best Mode for Carrying Out the Invention

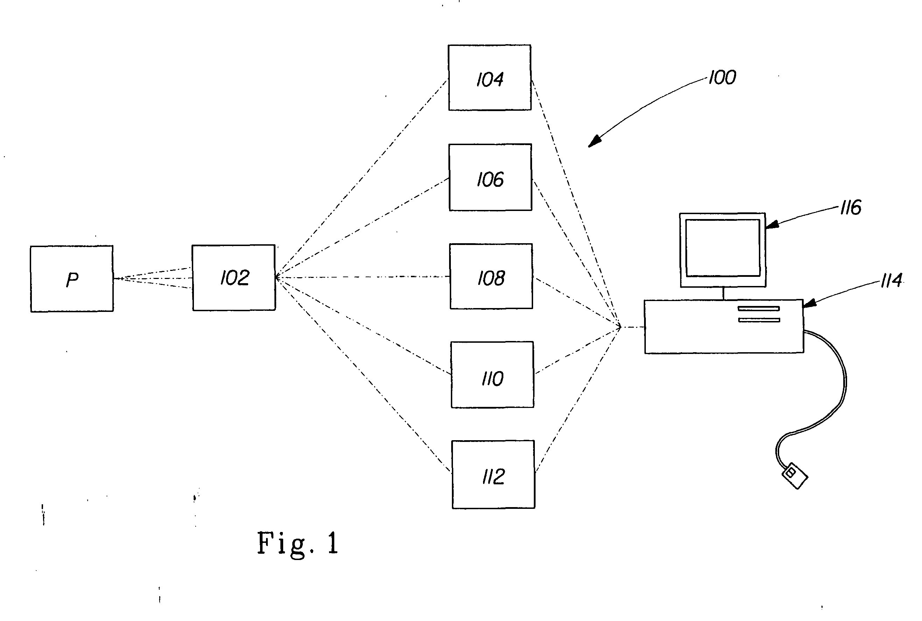

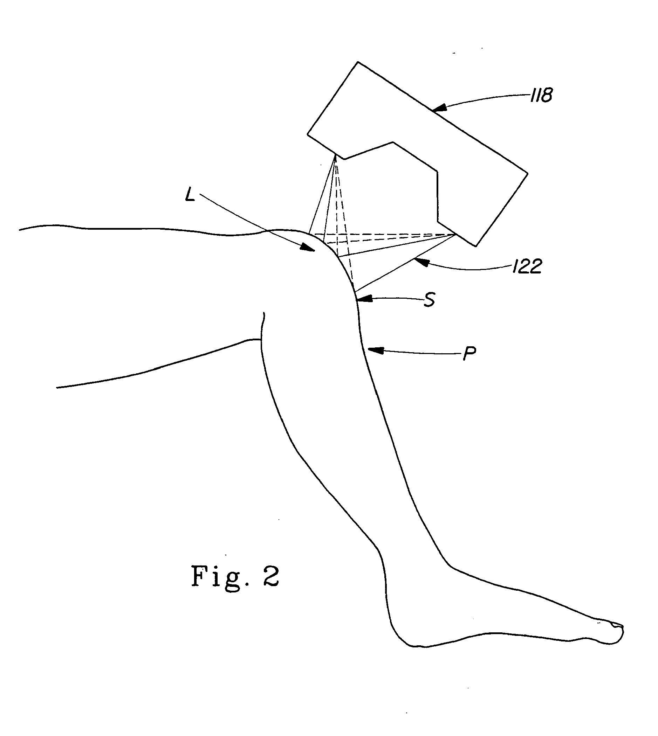

Referring to **FIGS. 1** and **2**, a non-human observer or non-clinician apparatus for the detection and quantification of joint and tissue inflammation, generally designated **100,** is shown comprising a sensing component **102** for obtaining and collecting data indicative of the surface at the location **L** of the joint or tissue of a patient **P.** Preferably, the sensing component **102** comprises various components for assessing inflammation such as a device for detecting swelling **104,** such as an optical imaging system, by providing dimensional measurement of the location **L** of the joint or tissue of the patient **P** being examined for inflammation, a range-of-motion device **106** for determining the patient's range of motion of the joint being examined, a color analyzing device **108** for analyzing color of the patient's skin at the location **L** of the joint or tissue, a temperature measuring device **110** for measuring the temperature of the skin **S** of the patient **P** at the location **L** of the joint or tissue, a pain detection device **112** for determining the threshold and tolerance of pain, an archival storage and retrieval device **114,** such as a computer, for storing data being collected and for correlating and analyzing, and a display device **116** for viewing the analyzed data.

Referring to **FIG. 2,** a patient **P** being tested for joint and tissue inflammation is shown positioned such that the location **L** of the tissue or joint to be examined is appropriately positioned for measurement by the apparatus **100** of the present invention. The sensing component **102** includes a surface scanning element **118** having a digitizing scanner which is coupled for use with the device for detecting swelling **104** for dimensional measurement of the location **L** of the tissue or joint being examined for inflammation. Preferably, the surface scanning element **118** may comprise a flat bed scanner for providing two-dimensional images and measurement data of the location being examined, or multiple scanners, such as are used currently in the manufacture of high precision casting and machine components, for example, suitably oriented for providing three-dimensional images and measurement data of the area being examined. The device for detecting swelling **104** may also comprise various thermography devices, such as infrared imaging systems used for security identification, infrared inspection of electrical and mechanical components, or medical imaging. The device for detecting swelling **104** may also comprise high-resolution ultrasound or magnetic resonance systems, such as used in medical imaging.

Referring to **FIGS. 1** and **2,** an example of the preferred embodiment of the invention is shown whereby the device for detecting swelling **104** is a conventional video-based digitizing scanning system which is coupled to the surface scanning element **118** of the sensing component **102.** The surface scanning element **118** operates to collect data and transfer the data to the device for detecting swelling **104,** such as by the use of an analog to a digital converter (not shown), which is coupled to the archival storage and retrieval device **114** that operates to store and analyze the collected data to generate measurements, and preferably a mathematical model or image indicative of the surface and cross-sectional dimensions of the joint or tissue at the location **L.** The specific measurements and the mathematical model or image, if any, can be fed to the display device **116** for viewing.

In another preferred embodiment of the invention, the device for detecting swelling **104** is a conventional laser-based digitizing scanning system which is coupled to the surface scanning element **118** of the sensing component **102.** The device **104** includes means for moving a light beam **122,** such as a laser beam emitted from the surface scanning element **118** across the surface area of the location **L** being investigated. The surface scanning element **118** collects data and transfers the data to the device for detecting swelling **104** which is coupled to the archival storage and retrieval device **114** that operates to store the collected data and to analyze the pattern of reflections and the characteristics of the beam spot pattern of reflections from the scanned location **L** to generate a model or image indicative of the surface and cross-sectional dimensions of the joint or tissue at the location **L.** The archival storage and retrieval device **114** then feeds the generated model or image, if any, and pertinent measurements to the display device **116** for viewing.

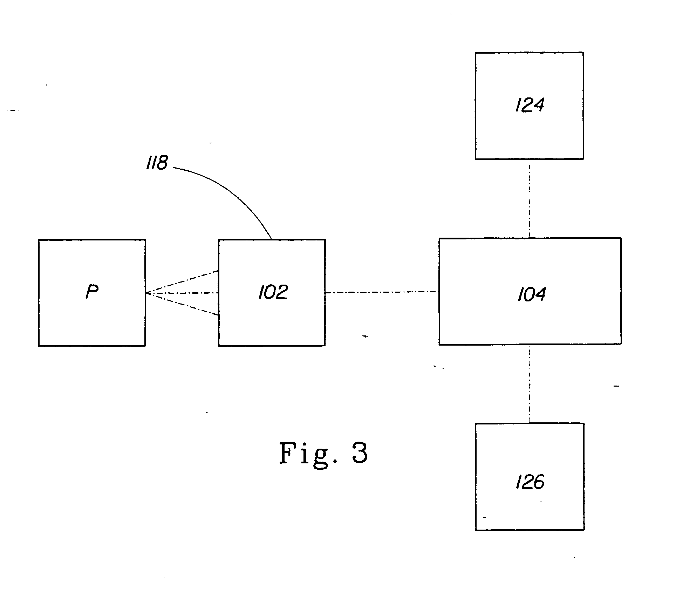

In another preferred embodiment of the present invention, the device for detecting swelling **104** is a conventional position tracking based scanning system which is coupled to the sensing component **102** having a hand-held, free-motion surface scanning element **118.** Preferably, the surface scanning element **118** is manually moved about the location **L** to be scanned by an operator. Referring to **FIG. 3,** the imaging system **104** is shown having a distance measuring device **124** to measure the distance between the surface scanning element **118** and the surface at the location **L** being scanned and a position tracking device **126** to detect the position and orientation of the surface scanning element **118** within a position reference field. In operation, the device for detecting swelling **104** tracks the location of the surface scanning element **118** and is conventionally coupled to the archival storage and retrieval device **114** which receives location data from the device **104** and correlates and generates the cross-sectional dimensions, and preferably an image, of the scanned joint or tissue at the location **L.** The archival storage and retrieval device **114** then feeds the pertinent measurements and the generated image, if any, to the display device **116** for viewing.

In another preferred embodiment of the present invention, the device for detecting swelling **104** is a conventional position tracking based scanning system which is coupled to the sensing component **102** having a free-motion digitized surface scanning element **118.** The position tracking system **126** detects the position of the surface scanning element **118** relative to a position reference magnetic field generated by a transmitter (not shown). In operation, the tissue or joint at the location **L** is measured by pressing the surface scanning element **118** against the skin **S** of the patient **P** and moving the surface scanning element **118** over the surface of the joint or tissue to be scanned. The position tracking system **126** tracks the position and orientation of the surface scanning element **118** as it moves over the location **L** and is coupled to the archival storage and retrieval device **114** which receives and correlates data and generates the cross-sectional dimensions, and preferably an image, of the joint or tissue at the scanned location **L.** The archival storage and retrieval device **114** then feeds the pertinent measurements and generated model or image, if any, to the display device **116** for viewing.

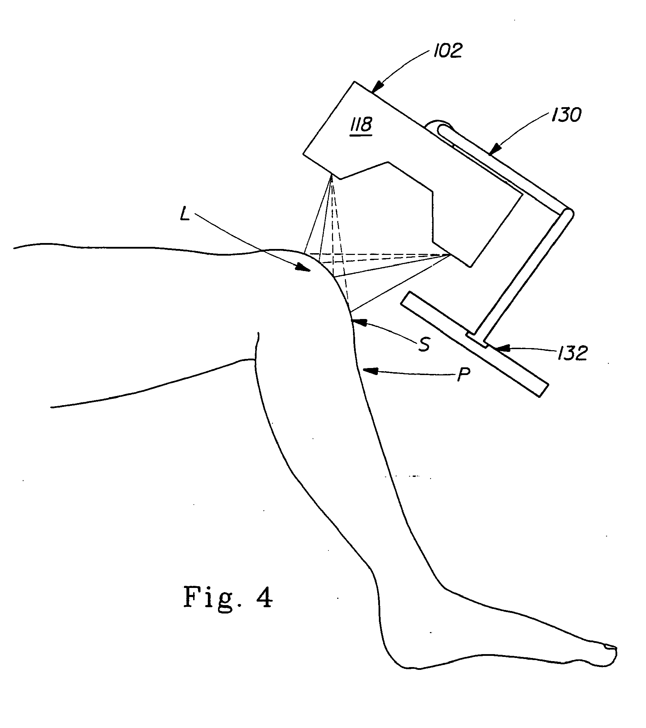

Another preferred embodiment of the present invention, as shown in **FIGS. 1** and **4,** the device for detecting swelling **104** is a conventional position based tracking system and is coupled to the sensing component **102** having a non-contact, restricted motion surface scanning element **118.** The device for detecting swelling **104** includes a support structure **130** for supporting the sensing component **102** and a guide track **132** for directing the motion of the sensing component **102.** A conventional brace (not shown) may be provided to hold the limb or other anatomic feature of the patient **P** being scanned in place during scanning. Alternatively, the sensing component **102** may also be attached to a mechanical arm (not shown) for directing its motion over the area to be scanned. In a preferred embodiment of the invention, the distance measuring device **124 (FIG. 3)** is a non-contact probe, such as a laser distance measuring device, that measures the displacement between the surface scanning element **118** and the skin **S** of the patient **P** at the location **L** being scanned. The position of the surface scanning element **118** is directed by a position tracking system **126 (FIG. 3)** by denoting the position of the surface scanning element **118** on the guide track **132** or the mechanical arm (not shown). In operation, the tissue or joint of the patient **P** is secured in place and the surface scanning element **118** is moved along the guide track **132** to scan the tissue or joint. The device for detecting swelling **104** tracks the position of the surface scanning element **118** and is conventionally coupled to the archival storage and retrieval device **114** which analyzes the scanned data and the position of the surface scanning element **118** and generates an image and cross-sectional dimensions of the tissue or joint at the scanned location **L** which is fed to the display device **116** for viewing.

Another preferred embodiment of the present invention, as shown in **FIGS. 1, 2, 3,** and **4**, the device for detecting swelling **104** is a high-resolution ultrasound device and is coupled to the sensing component **102** having an ultrasound emitter and receiver scanning element **118.** The ultrasound emitter and receiver scanning element **118** may be a hand-held, free-motion surface scanning device **(FIG. 2)** or a restricted-motion scanning element utilizing a support structure **130** and a guide track **132 (FIG. 4).** A conventional brace (not shown) may be used to hold the limb or other anatomic feature of the patient **P** being scanned during the scanning operation. A position tracking device **126** may be attached to the device for detecting swelling **104 (FIG. 3)** to determine the position and orientation of the ultrasound probe **118** within a position reference field. In operation, ultrasonic beams from the scanning element **118** are emitted through and then reflected back from the tissue of the patient **P** at the location **L.** The nature of, degree of, and the rapidity of the reflected ultrasound signal detected by the receiver sensor of the surface scanning element **118** are transmitted to the device for detecting swelling **104** which is coupled to the archival storage and retrieval device **114.** The archival storage and retrieval device **114** conventionally analyzes these data and generates the cross-sectional dimensions, preferably a model or image, of the scanned joint or tissue area at the location **L,** and this is fed to the display device **116** for viewing. Additionally, the nature and characteristics of the reflected ultrasound signal detected by the receiver sensor of the surface scanning element **118** and transmitted by the device for detecting swelling **104** to the archival storage and retrieval device **114** could be analyzed using a separate analyzer (not shown), such as a computer or a program, to determine the location and degree of inflammation via various properties, such as the particular echogenicity, of the reflected ultrasonic signal.

In another preferred embodiment of the present invention, as shown in **FIGS. 1, 3,** and 4, the device for detecting swelling **104** is a conventional magnetic resonance imaging (MRI) system. The sensing component **102** is a conventional magnetic resonance imaging coil and preferably is a restricted motion scanning element using a support structure **130** and a guide track structure **132 (FIG. 4).** A conventional brace (not shown) may be used to hold the limb or other anatomic feature of the patient **P** being scanned. The magnetic resonance field is conventionally oriented and located in a reference spatial grid as with all conventional MRI devices. In operation, magnetically generated images or dimensions from the anatomy of the patient **P** at the location **L** are transmitted from the surface scanning element **118** to the device for detecting swelling **104** and this in turn is conventionally coupled to the archival storage and retrieval device **114.** The archival storage and retrieval device **114** operates to conventionally analyze these MRI data and generate the cross-sectional dimensions, and preferably a model or image, of the area of the scanned joint or tissue at the location **L.** These analyzed data are then fed to the display device **116** for viewing. Additionally, the nature and characteristics of the returned MRI signal detected by the surface scanning element **118** can be analyzed by the archival storage and retrieval device **114** to determine the location and degree of inflammation via various properties of the MRI signal.

It should now be apparent to those skilled in the art that the imaging system **104** of the subject invention can be formed from various types of conventional dimensional measuring or imaging systems that are capable of producing detailed images and/or measurement of surfaces. Preferably, such systems should be such that they are easily adapted for use in developing detailed measurements, and preferably computerized models or images, of the joints or tissue. It should also now be apparent to those skilled in the art that other types of systems, such as wraps, cuffs, or sleeves having one or a plurality of location sensors may be used for placement about a joint or tissue area.

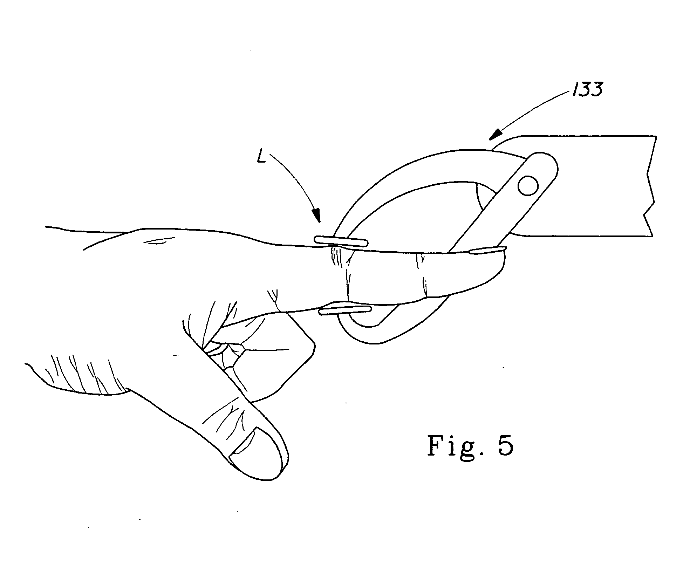

In another preferred embodiment of the invention, as shown in **FIGS. 2** and **5,** the imaging system **104** comprises mechanical calipers **133.** In operation, the mechanical calipers **133** are applied to directly measure the cross-sectional dimensions or elevations of the joint or tissue at the location **L.** The position of the calipers **133** is determined by the position tracking system **126.** The calibers **133** and the position tracking system **126** are coupled to the archival storage and retrieval device **114** for archival storage and retrieval and for correlating the data and generating the cross-sectional dimensions, and preferably a three-dimensional image, of the joint or tissue at the location **L.**

It should now be apparent to those skilled in the art that the model(s) or image(s), if any, and the cross-sectional dimensions generated using the apparatus for the detection and quantification of joint and tissue inflammation **100** of the subject invention may be compared to a reference, such as a previously taken dimensional measurements and/or models or images of the location **L,** and slight variations in the position of the surface of the location **L** of the tissue or joint can be observed, thus allowing precise determination of any differences or changes of the subject joint or tissue cross-sectional area or dimensions.

Referring to **FIGS. 1** and **2,** the device for detecting swelling **104** preferably also operates as the range-of-motion device **106** of the present invention for determining the patient's range of motion of the joint being examined. Using the dimensional measurements and/or the images, if any, generated by the device for detecting swelling **104,** the range-of-motion of the joint may be determined using the archival storage and retrieval device **114** to measure the changes in the position and deformation of certain pre-selected points within the location **L** being scanned as the joint is moved. The measurements can then be compared to a reference, such as a standard or the results of a previous scan, to determine any changes in the range of motion of the subject joint. While the range-of-motion device **106** preferably uses the images or measurements generated by the device for detecting swelling **104,** it should now be apparent to those skilled in the art that other range-of-motion devices such as those using geometric charts and gages, optical grids, or those using mechanical systems known in the art for measuring ranges of motion may also be used and the results stored in the archival storage and retrieval device **114** for archival storage and retrieval and for correlating and analyzing the data and for generating a graphical illustration or measurement of the patient's range-of-motion. These data and the graphical illustrations can be fed into a display device **116** for viewing.

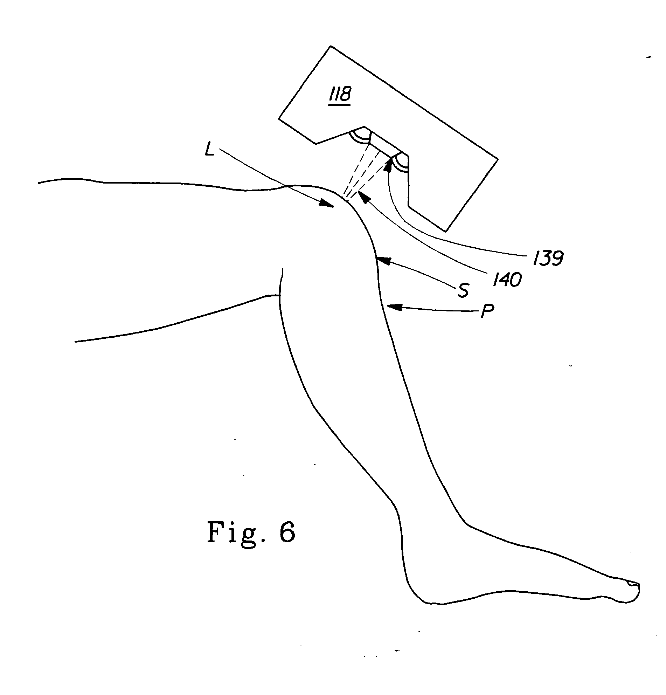

The color analyzing device **108** for analyzing the color of the skin or tissue of the patient **P** at the location **L** of the tissue or joint being examined is shown in **FIGS. 1** and **6.** The color analyzing device **108** is coupled to the surface scanning element **118** of the sensing component **102,** which preferably comprises a light-sensitive sensor for measuring color such as a colorimeter or a spectrophoto-meter. In operation, a patient **P** being tested for joint or tissue inflammation is arranged in such a manner that the tissue or joint to be examined is appropriately positioned for measurement by the apparatus of the present invention. The surface scanning element **118** is directed over the subject location **L** and the measurement of the wavelength and the intensity of the electromagnetic radiation being reflected from the location **L** is measured using the color analyzer of the color analyzing device **108.** The color analyzing device **108** is coupled to the archival storage and retrieval device **114** for archival storage and retrieval and for comparing the data from the color analyzing system **108** to a standard or to a reference, such as previously taken measurements of the subject location **L** (or measurements of other noninflamed anatomical locations), for determining an increase or a decrease in inflammation. These data are then transmitted to the display device **116** for viewing.

In another preferred embodiment of the invention, the color analyzing device **108** is coupled to the surface scanning element **118** of the sensing component **102.** Preferably, the surface scanning element **118** includes a light sensitive sensor, i.e. a glossmeter, for measuring the surface characteristics of the subject location **L.** In operation, photoelectric measurement of specularly reflected light from the surface of the skin **S** of the patient **P** at the location **L** is measured by the color analyzing device **108.** The color analyzing device **108** includes a gloss analyzer for comparing the collected data to a standard, a reference such as a previous measurements of the location **L,** or to measurements of other noninflammed anatomical locations, and is coupled to the archival storage and retrieval device **114** for archival storage and retrieval of the collected data and for comparing data from the color analyzing device **108** to a standard or to a reference, such as a previous taken measurements of the subject location **L,** or measurements of other noninflammed anatomical locations for determining an increase or decrease in inflammation. Additionally, the gloss reading from the surface scanning element **118** of the sensing component **102** could be fed to the device for detecting swelling **104** and then transmitted to the archival storage and retrieval device **114** which would analyze, or use an attached element or program (not shown) to analyze the gloss reading to determine the tissue stretch and thus the associated swelling of the joint or tissue at the location **L** of the patient **P.** These color or gloss data are then transmitted to the display device **116** for viewing.

Referring to **FIGS. 1**, **2,** and **6,** the temperature measuring device **110** for measuring the temperature in the location **L** of the tissue or joint to be examined is shown and is coupled to the sensing component **102** having a surface scanning element **118** for receiving temperature input of the subject location **L.** In a preferred embodiment of the invention, the surface scanning element **118** includes a light-sensitive sensor for receiving temperature input by measuring the intensity of an infrared light beam **140** which is reflected from the surface of the skin or tissue **S** of the patient **P** at the subject location **L.** Infrared sensing devices have been commercially available for measuring skin temperatures of patients. In a preferred embodiment of the invention, as shown, the surface scanning element **118** of the temperature measuring device **110** includes a conventional infrared sensor comprising an infrared detector **138** and a focusing element **139** for focusing incoming infrared radiation emitted from the skin or tissue **S** of the patient **P** onto the infrared detector of the surface scanning element **118.** Broadcast radiation is detected by the surface scanning element **118** and the temperature is calculated using conventional circuitry within the temperature measuring device **110** that calculates subtle skin or tissue temperature variations. The temperature measuring device **110** correlates the data and compares the data to a standard or a reference, such as previous temperature measurements at that location **L,** and the difference in the intensity of the infrared light can be used to calculate the temperature and the change in temperature of the scanned location **L.** The temperature measuring device **110** is coupled to the archival storage and retrieval device **114** for calculating temperature changes and for archival storage and retrieval of the temperature data and for comparing the data to a standard or to a reference, such as previously taken measurements of the subject location **L,** for determining an increase or a decrease in inflammation. The archival storage and retrieval device **114** feeds the changes to the display unit **116** for viewing.

While preferably the surface scanning element **118** includes a light-sensitive sensor, other forms of sensors may also be utilized for measuring the skin or tissue **S** temperature of the patient **P.** In a preferred embodiment of the invention, the surface scanning element **118** comprises a conventional thermocouple temperature sensor whereby pairs of dissimilar metal alloy wires join at least one end, which generate a net thermoelectric voltage between the two ends according to the size of the temperature difference between the ends, the relative Seebeck coefficient of the wire pair and the uniformity of the wire's relative Seebeck coefficient.

In another preferred embodiment of the invention, the surface scanning element **118** comprises a conventional thermistor temperature sensor having electric contacts and lead conductors connected to the contact whereby the induced specific resistance across the thermistor is measured and converted to a specific temperature reading using conventional circuitry.

In another preferred embodiment of the invention, the surface scanning element **118** comprises a conventional resistance temperature detector whereby electrical current that produces a voltage drop across the sensor is measured and is converted to a temperature reading using conventional circuitry.

In another preferred embodiment of the invention, the surface scanning element **118** comprises a conventional pulsed laser beam temperature sensor whereby a first laser beam having a first wavelength is oscillated immediately after the rise of the pulsed laser beam, and a second laser beam having a second wavelength is oscillated thereafter. Based on the difference between the intensity of the first interfered light of reflected light of the first laser beam and the intensity of reflected light of the second laser beam, the temperature of the skin **S** of the patient **P** can be measured.

In another preferred embodiment of the invention, the surface scanning element **118** includes a conventional skin surface electrical conductance/resistance probe, similar to those in polygraph systems, comprising two electrodes which are placed in direct contact with the skin of the patient. In operation, the electrodes are provided with electric current that flows between them and changes in the conductance/resistance of the flow caused by insensible sweating at the location is measured. Since tissue temperature changes cause variations in insensible sweating and thus skin moisture, changes in skin electrical conductance/resistance occur and from these changes the temperature at the location of the probe can be derived using the archival storage and retrieval device 114.

It should now be apparent to those skilled in the art that the temperature measuring device **110** of the subject invention can be formed from various types of conventional temperature measuring devices that are capable of measuring the temperature of a surface Such devices may include conventional medical thermography instrumentation, microwave thermography instrumentation for measuring natural radiation, infrared scanners, laser scanners, thermometers, resistance temperature detectors, thermistor temperature sensors, and thermocouple temperature sensors. Preferably, such devices should be easily adapted for use in developing detailed measurements of the temperature along the surface of the area of the joint or tissue being examined.

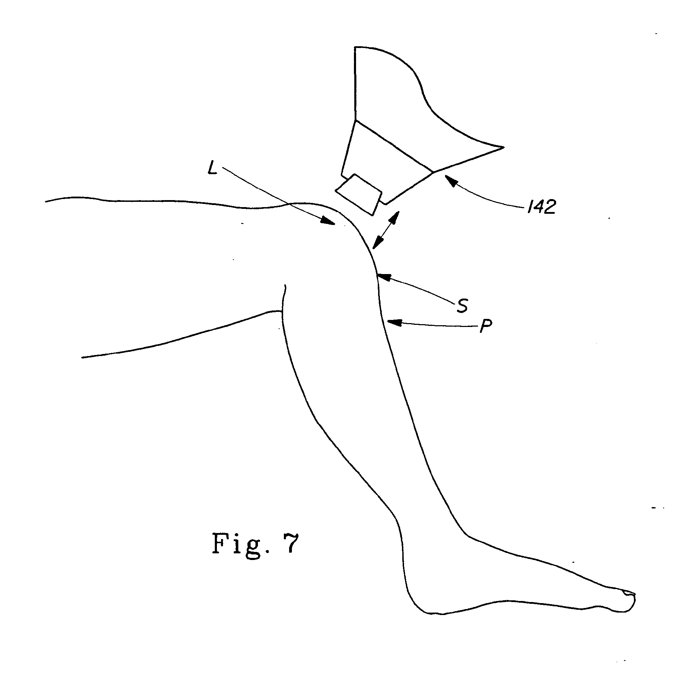

Referring to FIGS. 1 and 7, the pain or tenderness detection device 112 for determining the presence of pain or tenderness is shown. In a preferred embodiment of the invention, the sensing component 102 includes a probe 142, such as a hand-held pressure probe, e.g. a dolorimeter, to determine pain or tenderness threshold and tolerance of the patient P at the location L. In operation, the probe 142 comprises a pressing device, clamping device, sleeve, pinchers, or other like means for applying pressure against the skin S of the patient P at the location L being examined. Preferably, the pressure level necessary to produce pain or tenderness at the location L is sensed by the probe 142 via a button or other conventional triggering device (not shown) which the patient P activates when the applied pressure stimulus becomes painful, and this pressure level is then coupled to the to the archival storage and retrieval device 114 for converting the readings of the probe 142 into a graphical representation.

In another preferred embodiment of the invention, the sensing component 102 includes a probe **142** for producing a discrete area of heat. The probe **142** comprises a metal tip which is easily heated electrically. The heat level necessary to produce pain at the location **L** is determined via a button or other triggering device (not shown) which the patient **P** activates when the applied heat becomes painful, and this heal level is then fed to the pain detection device **112** which calculates the temperature at which the patient **P** experiences pain at the location **L** and this is transmitted to the an archival storage and retrieval device **114** for storage and retrieval of generated data and for analysis and is fed into the display **116** for viewing.

In another preferred embodiment of the invention, the sensing component **102** includes a probe **142,** for producing discrete electrical stimuli. The probe **142** comprises a metal tip which is effective for electrically stimulating the tissue of the patient **P** at the location **L.** The electrical stimulus necessary to produce pain at the location **L** is determined via a button or other triggering device (not shown) which the patient **P** activates when the applied electrical stimulus becomes painful, and this stimulus level is then fed to the pain detection system **112** which then calculates the amount of electrical stimuli to determine the pain threshold and tolerance at the location **L** and this is transmitted to the archival storage and retrieval device **114** for archival storage and retrieval and for analysis and is fed into the display **116** for viewing.

It should now be apparent to those skilled in the art that pain and tenderness thresholds and tolerances will vary significantly between patients, and such quantified results together with visual observations will significantly improve reliability in assessing a patient's pain and tenderness thresholds and tolerances.

It should now be apparent to those skilled in the art that the present invention provides a novel non-human observer or non-clinician based apparatus and method for the detection and quantification of joint or tissue inflammation comprising an imaging or measuring system for obtaining data indicative of the surface and cross-sectional dimensions of the patient's tissue or skin, a color and/or gloss analyzing device for analyzing the color and/or gloss of the patient's tissue or skin, a temperature measuring device for measuring the temperature of the patient's tissue or skin, a pain detection device for determining the patient's threshold of tenderness or pain; and a device for determining the patient's range of motion at the location being examined. Accordingly, any two or more of the five cardinal signs or manifestations comprising redness, swelling, heat, pain, and the loss of function of the involved tissue, common to inflammation, can be recorded and analyzed by the examining physician, trainer, or health care worker. The presence of inflammation in the involved joint or tissue can be used as an indication of the presence of injury or disease, while the amount of inflammation in the injured, deformed, or arthritic joint or tissue can be used to determine the amount of damage or disease in that joint and whether the problem is progressing or healing.

It should be understood that the various groups of measurements of swelling, color, temperature, function or range-of-motion, and pain obtained using the apparatus for detecting and quantifying inflammation of a joint or tissue area of the subject invention will be used to detect and quantify the location and the amount of joint or tissue inflammation. It should now be apparent to those skilled in the art that mathematical algorithms or models may be developed to yield an overall inflammation quantity or score which can be used to assess the location and the degree of inflammation and any changes from baseline or previous measurements of inflammation.

It should now be apparent that the non-human observer or non-clinician based apparatus and method for the detection and quantification of joint and tissue inflammation of the subject invention eliminates observer subjectivity and interindividual variation and thus providing better accuracy and reproducibility in the measurement of the detection and quantification of joint and tissue inflammation.

It should also now be apparent to those skilled in the art that the present invention provides a reliable and reproducible means whereby researchers can develop algorithms based on clinical studies that may be used to more accurately identify and quantify inflammation.

It should also now be apparent to those skilled in the art that the present invention provides a relatively inexpensive, reliable, reproducible, easy to use or perform, safe and noninvasive method and apparatus for the detection and quantification of joint and tissue inflammation that may be used in medical offices, clinics, training and sports facilities, and the like.

It should also now be apparent to those skilled in the art that the method and apparatus for the detection and quantification of joint and tissue inflammation can also comprise operating instructions. Such instructions can be in the form of computer software stored within the computer, printed material attached to the apparatus or in the form of brochures or books.

## Claims

1. An apparatus (100) for detecting and quantifying inflammation of a joint or tissue area comprising: a sensing component (102) for obtaining quantitative, non-human-observer measurements of at least two of the cardinal signs of inflammation; a device (114) for storing the measurements; a display device (116) for displaying the measurements; and means for analyzing collected measurements and generating an overall inflammation score.

2. The apparatus of Claim 1 further comprising means for comparing the measurements with previous measurements of the joint or tissue area.

3. The apparatus of Claim 1 wherein said sensing unit comprises a device (104) for detecting swelling of the joint or tissue area.

4. The apparatus of Claim 3 wherein said device (104) for detecting swelling is selected from the group comprising mechanical contact devices, video-based digitizing scanning system devices, optical scanning system devices, laser-based system devices, position tracking based system devices, glossmeter-based devices, ultrasound devices, and magnetic resonance imaging and measuring system devices.

5. The apparatus of Claim 1 wherein said sensing component comprises a range-of motion device (106) for determining the range of motion of the joint or tissue.

6. The apparatus of Claim 5 wherein said range-of-motion device (106) is selected from the group comprising geometric chart devices, geometric gage devices, optical grid devices, video-based digitizing scanning system devices, optical scanning system devices, laser-based system devices, position tracking based system devices, ultrasound-based devices, and magnetic resonance imaging and measuring system devices.

7. The apparatus of Claim 1 where said sensing component comprises a color analyzing device (108) for analyzing the color of the area of the joint or tissue area.

8. The apparatus of Claim 7 wherein said color analyzing device (108) is a light-sensitive sensor device.

9. The apparatus of Claim 7 wherein said color analyzing device (108) is selected from the group comprising colorimeter devices, spectrophotometer devices, and glossmeter devices.

10. The Apparatus of Claim 1 wherein said sensing component comprises a temperature
measuring device (110) for measuring the temperature of the joint or tissue area.

11. The Apparatus of Claim 10 wherein said temperature measuring device is selected
from the group comprising mechanical contact devices, optical devices, laser-based devices, thermistor type devices, thermocouple-type devices, thermometer devices, thermography-type devices, infrared-based devices, light sensitive devices, and surface electrical conductance-resistance based devices.

12. The Apparatus of Claim1 further comprising means (114) for generating an image of the joint or tissue area.

13. The apparatus of Claim 1 further comprising means (114) for generating a mathematical model of the joint or tissue area.

## Patentansprüche

1. Gerät (100) zum Detektieren und Quantifizieren einer Entzündung eines Gelenk- oder Gewebebereichs, umfassend: eine erfassende bzw. Abtastkomponente (102) zum Erhalten von quantitativen nicht von einem menschlichen Beobachter stammenden Messungen von wenigstens zwei der grundlegenden Zeichen bzw. Anzeichen einer Entzündung; eine Vorrichtung (114) zum Speichern der Messungen; eine Anzeigevorrichtung (116) zum Anzeigen der Messungen; und Mittel zum Analysieren von gesammelten Messungen und Generieren bzw. Erzeugen einer Gesamtentzündungszahl bzw. menge.

2. Gerät nach Anspruch 1, weiterhin umfassend Mittel zum Vergleich der Messungen mit vorherigen Messungen des Gelenk- oder Gewebebereichs.

3. Gerät nach Anspruch 1, wobei die Erfassungs- bzw. Abtasteinheit eine Vorrichtung (104) zum Detektieren einer Schwellung des Gelenk- oder Gewebebereichs umfaßt.

4. Gerät nach Anspruch 3, wobei die Vorrichtung (104) zum Detektieren einer Schwellung gewählt ist aus der Gruppe, umfassend mechanische Kontaktvorrichtungen, auf Video basierende digitalisierende Scan- bzw. Abtastsystemvorrichtungen, optische Scan- bzw. Abtastsystemvorrichtungen, auf Laser basierende Systemvorrichtungen, auf Positionsverfolgung basierende Systemvorrichtungen, auf einem Glanzmeßgerät basierende Vorrichtungen, Ultraschallvorrichtungen und Magnetresonanzabbildungs- und meßsystemvorrichtungen.

5. Gerät nach Anspruch 1 , wobei die Abtastkomponente eine Bewegungsbereichvorrichtung (106) zum Bestimmen des Bereichs einer Bewegung des Gelenks oder Gewebes umfaßt.

6. Gerät nach Anspruch 5, wobei die Vorrichtung (106) betreffend den Bereich der Bewegung gewählt ist aus der Gruppe, umfassend geometrische Tafel- bzw. Kartenvorrichtungen, geometrische Lehrenvorrichtungen, optische Gittervorrichtungen, auf Video basierende digitalisierende Scan- bzw. Abtastsystemvorrichtungen, optische Scan- bzw. Abtastsystemvorrichtungen, auf Laser basierende Systemvonichtungen, auf Positionsverfolgung basierende Systemvorrichtungen, auf Ultraschall basierende Vorrichtungen und Magnetresonanzabbildungs- und -meßsystemvorrichtungen.

7. Gerät nach Anspruch 1, wobei die Abtastkomponente eine Farbanalysiervorrichtung (108) zum Analysieren der Farbe des Bereichs des Gelenk- oder Gewebebereichs umfaßt.

8. Gerät nach Anspruch 7, wobei die Farbanalysiervorrichtung (108) eine lichtempfindliche Sensorvorrichtung ist.

9. Gerät nach Anspruch 7, wobei die Farbanalysiervorrichtung (108) gewählt ist aus der Gruppe, umfassend Colorimeter-Vorrichtungen, Spektrophotometa-Vorrichtungen und Glanzmeßvorrichtungen.

10. Gerät nach Anspruch 1, wobei die Abtastkomponente eine Temperaturmeßvorrichtung (110) zum Messen der Temperatur des Gelenk- oder Gewebebereichs umfaßt,

11. Gerät nach Anspruch 10, wobei die Temperaturmeßvorrichtung gewählt ist aus der Gruppe, umfassend mechanische Kontaktvorrichtungen, optische Vorrichtungen, auf Laser basierende Vorrichtungen, Thermistortyp-Vorrichtungen, Thermoelement-Vorrichtungen, Thermometervorrichtungen, Thermographietyp-Vorrichtungen, auf Infrarot basierende Vorrichtungen, lichtempfindliche Vorrichtungen und auf elektrischem Oberflächen-Leitfähigkeits-Widerstand basierende Vorrichtungen.

12. Gerät nach Anspruch 1, weiterhin umfassend Mittel (114) zum Generieren eines Bilds des Gelenk- oder Gewebebereichs.

13. Gerät nach Anspruch 1, weiterhin umfassend Mittel (114) zum Generieren bzw. Erzeugen eines mathematischen Modells des Gelenk- oder Gewebebereichs.

## Revendications

1. Appareil (100) pour détecter et quantifier l'inflammation d'une zone d'articulation ou de tissus comprenant : un composant de détection (102) pour obtenir des mesures d'un observateur non-humain, quantitatives d'au moins deux des signes cardinaux de l'inflammation ; un dispositif (114) pour stocker les mesures ; un dispositif d'affichage (116) pour afficher les mesures ; et des moyens pour analyser les mesures recueillies et générer un résultat global d'inflammation.

2. Appareil selon la revendication 1, comprenant, en outre, des moyens pour comparer les mesures avec les mesures précédentes de la zone d'articulation ou de tissus.

3. Appareil selon la revendication 1, dans lequel ladite unité de détection comprend un dispositif (104) pour détecter une tuméfaction de la zone d'articulation ou de tissus.

4. Appareil selon la revendication 3, dans lequel ledit dispositif (104) pour détecter une tuméfaction est choisi dans le groupe comprenant les dispositifs à contact mécanique, les dispositifs à système de balayage numérique à base de vidéo, les dispositifs à système de balayage optique, les dispositifs à système à base de laser, les dispositifs à système basé sur le pistage de position, les dispositifs à base de brillancemètre, les dispositifs à ultrasons, et les dispositifs à système d'imagerie et de mesures par résonance magnétique.

5. Appareil selon la revendication 1, dans lequel ledit composant de détection comprend un dispositif à plage de mouvement (106) pour déterminer la plage de mouvement de l'articulation ou du tissu.

6. Appareil selon la revendication 5, dans lequel ledit dispositif à plage de mouvement (106) est choisi dans le groupe comprenant les dispositifs à diagramme géométrique, les dispositifs à jauge géométrique, les dispositifs à grille optique, les dispositifs à système de balayage numérique à base de vidéo, les dispositifs à système de balayage optique, les dispositifs à système à base de laser, les dispositifs à système basé sur le pistage de position, les dispositifs à base d'ultrasons, et les dispositifs à système d'imagerie et de mesures par résonance magnétique.

7. Appareil selon la revendication 1, dans lequel ledit composant de détection comprend un dispositif d'analyse de couleur (108) pour analyser la couleur de la zone de l'articulation ou de la zone de tissus.

8. Appareil selon la revendication 7, dans lequel ledit dispositif d'analyse de couleur (108) est un dispositif de détection sensible à la lumière.

9. Appareil selon la revendication 7, dans lequel ledit dispositif d'analyse de couleur (108) est choisi dans le groupe comprenant les colorimètres, les spectrophotomètres, et les brillancemètres.

10. Appareil selon la revendication 1, dans lequel ledit composant de détection comprend un dispositif de mesure de température (110) pour mesurer la température de la zone d'articulation ou de tissus.

11. Appareil selon la revendication 10, dans lequel ledit dispositif de mesure de température est choisi dans le groupe comprenant les dispositifs à contact mécanique, les dispositifs optiques, les dispositifs à base de laser, les dispositifs de type thermistors, les dispositifs de type thermocouples, les thermomètres, les dispositifs de type thermographes, les dispositifs à base d'infrarouges, les dispositifs sensibles à la lumière, et les dispositifs de surface à base de conductance-résistance électrique.

12. Appareil selon la revendication 1, comprenant, en outre, des moyens (114) pour générer une image de la zone d'articulation ou de tissus.

13. Appareil selon la revendication 1, comprenant, en outre, des moyens (114) pour générer une simulation mathématique de la zone d'articulation ou de tissus.
